# EUROPÄISCHE PATENTSCHRIFT

(11) **EP 0 965 841 B1**
(45) Veröffentlichungstag und Bekanntmachung des Hinweises auf die Patenterteilung: **14.03.2001**
(21) Anmeldenummer: 98106246.6
(22) Anmeldetag: 06.04.1998
(51) Int. Cl.: G01N 33/96

(54) **Kontroll- oder Kalibrierstandard für Messgeräte zur optischen Bestimmung der Hämoglobinkonzentration in Blutproben**
Control or calibration standard for instruments for the optical determination of hemoglobin concentration in blood samples
Référance liquide de contrôle ou d'étalonnage pour des appareils de mesure optique du taux d'hémoglobine dans des échantillons de sang

(43) Veröffentlichungstag der Anmeldung: 22.12.1999
(73) Patentinhaber: AVL Medical Instruments AG, 8207 Schaffhausen (CH)
(72) Erfinder: Ben-David, Daniel, Alpharetta, Georgia 30004 (US); Tusa, James K., Alpharetta, Georgia 30004 (US); Kroneis, Herbert, A-8052 Graz (AT); Laughinghouse, Charles L., Cumming, Georgia 30040 (US); Ziegler, Werner, A-8043 Graz (AT); Jöbstl, Ewald, 8052 Graz (AT)
(74) Vertreter: Babeluk, Michael, Dipl.-Ing. Mag.

(56) Entgegenhaltungen:
- EP-A- 0 774 655
- US-A- 4 945 062
- US-A- 5 187 100
- US-A- 5 547 874
- US-A- 5 605 837
- DATABASE WPI Week 9003 Derwent Publications Ltd., London, GB; AN 90-020045 XP002079169 KURIYAMA MASAKA ET AL.: "Standard liquid for calibration of haemo-cytometer - comprising spheres of 1.9-3 and 4.5-5.5 micron dia. with sulphonic acid or sulphonate gps. on surface in aq. dispersion" & JP 01 301166 A (JAPAN SYNTHETIC RUBBER CO LTD), 5. Dezember 1989
- DATABASE WPI Week 8846 Derwent Publications Ltd., London, GB; AN 88-328146 XP002079170 MORIYAMA SEIICHI ET AL.: "Standard liq. for calibration of haemo-cytometer - contg. dye with specified peak absorbence, for determining haemoglobin value" & JP 63 243879 A (NIPPON KODEN KOGYO)

## Beschreibung

Die Erfindung betrifft einen Kontroll- oder Kalibrierstandard für Meßgeräte zur optischen Bestimmung der Hämoglobinkonzentration in Blutproben, welcher in einer wäßrigen Elektrolytlösung dispergierte Streukörper aufweist.

Für die Kalibrierung und Qualitätskontrolle medizinischer Meßgeräte, insbesondere zur Bestimmung der Konzentration unterschiedlicher Blutbestandteile ist es notwendig, stabile lagerfähige Kontroll- und Kalibrierstandards bereitzustellen, mit welchen derartige Meßgeräte vor der Messung kalibriert und/oder in gewissen Zeitintervallen einer Qualitätskontrolle unterzogen werden können.

Bekannte Hämoglobinstandards basieren beispielsweise auf einer Suspension von roten Blutzellen und Blutplättchen in einer Elektrolytlösung, welche zur Bestimmung der Teilchenzahl, des Hämatokrit und der Hämoglobinkonzentration verwendet werden. Bei der Herstellung des Standars werden stabilisierte Zellbestandteile des Blutes verwendet. Ein derartiger Standard ist beispielsweise aus der GB 2 023 287 A bekannt.

Weiters ist es auch bekannt, Hämoglobinstandards aus lysierten Erythrozyten herzustellen, wobei diese mit destilliertem Wasser versetzt werden. Die überstehende Flüssigkeit wird mit einem Bakterizid versehen und gefriergetrocknet. Vor Gebrauch muß der Standard wieder mit Wasser versetzt werden, um die Ausgangslösung herzustellen. Ein derartiger Standard wird beispielsweise in der GB 1 129 873 A beschrieben.

Nachteilig bei diesen Standards ist die begrenzte Lagerfähigkeit sowie im Falle der gefriergetrockneten Standards die aufwendige Handhabung und die dabei nicht auszuschließenden Handhabungsfehler.

Aus der JP 01-301166 ist eine Standardflüssigkeit für die Kalibration eines Hämocytometers bekannt geworden, welche in einer wäßrigen Elektrolytlösung erste und zweite, im wesentlichen sphärische Partikel aufweist. Die Partikel tragen an der Oberfläche Sulfonsäuregruppen und/oder Sulfonatgruppen. Die ersten Partikel weisen eine durchschnittliche Größe von 1,9 bis 3µm, vorzugsweise 2 bis 2,9µm auf und entsprechen der Größe der Blutplättchen im menschlichen Blut, die zweiten Partikel weisen einen Durchmesser von 4,5 bis 5,5µm auf und korrespondieren mit der Größe der Erythrozyten. Über die bekannte Anzahl der Partikel pro Volumseinheit der Standardflüssigkeit kann der Teilchenzähler im Hinblick auf die zu bestimmende Zahl der Erythrozyten, Blutplättchen, u. s. w. kalibriert werden. Die Herstellung der Partikel erfolgt beispielsweise durch Polymerisation aus einem Vinyl-Monomer mit Sulfonsäuregruppen oder durch Copolymerisation des Monomers mit anderen Monomeren. Die Elektrolytlösung enthält z. B. Natriumchlorid, Kaliumchlorid und Natriumphosphat. Die Zahl der ersten Partikel zu jenen der zweiten verhält sich wie 1:10-40. Weiters kann die Standardflüssigkeit gefärbt werden, um als Standard für die Hämoglobinmessung im Blut verwendet zu werden.

Weiters ist in der US 4 945 062 A ein flüssiger Kontroll-Standard für die Qualitätskontrolle im Zusammenhang mit Blutanalysesystemen geoffenbart. Die Kontrollflüssigkeit eignet sich für Systeme, welche die Werte für pH, PCO₂ und PO₂ im Blut bestimmen. Weiters dient die Flüssigkeit auch als Kontrollstandard für ionenselektive Elektroden (ISE), insbesondere zur Bestimmung von ionsisiertem Kalzium, Geamtkalzium sowie der Na, K und Li-Ionenkonzentration in Blutproben. Zur Messung der Hämoglobinkonzentration (tHb) sowie verschiedener Hämoglobinfraktionen weist der autoclavierbare, stabile, homogene Standard ein Absorptionsmittel vorzugsweise Farbstoffe aus der Gruppe Acid Red Dye, Ponceau 3R Red Dye, Acid Yellow Dye and Acid Blue Dye auf.

Nachteilig bei den genannten Standards ist die Tatsache, daß die Partikel zum Teil sedimentieren und die Standards vor der Anwendung erst geschüttelt oder homogenisiert werden müssen, bzw. daß die zugesetzten Farbstoffe zum Teil mit sensitiven Schichten von Sensoren und Elektroden in den Meßgeräten nicht verträglich sind bzw. die damit erfaßten Meßwerte verfälschen können. Weiters sprechen partikelsensitive Meßeinrichtungen auf die zugesetzten Farbstoffe nicht an.

Aus der US 5,547,874 A ist ein Kontroll- oder Kalibrierstandard bekannt, welcher in einer wäßrigen Dispersion eine bestimmte Menge einer speziellen Substanz (particular substance) aufweist, welche gleich oder ähnlich jener Substanz ist, welche analysiert werden soll. Unter anderem wird ein Standard zur Bestimmung der Hämoglobinkonzentration angeführt, welcher 0 bis 100 g/dl, vorzugsweise 5 bis 50 g/dl Hämoglobin enthält. Durch deren Gehalt an biologischen Substanzen entsprechen derartige Standards jenen, die bereits in der GB 2 023 287 bzw. GB 1 129 873 beschrieben wurden, wobei die selben Nachteile auftreten. Gemäß Tabelle 1, Spalte 4 der US 5,547,874 A ist es auch möglich, für einen Hämoglobinstandard Farbstoffe (red dyes) zu verwenden. Diese Variante entspricht dem aus der US 4,945,062 A bekannten Kontroll-Standard. Auch hier sind die zugesetzten Farbstoffe mit sensitiven Schichten von Sensoren und Elektroden in den Meßgeräten nicht verträglich.

Aus der EP 0 774 655 A ist ein Standard für ein Zytometer bekannt, bei welchem ähnlich wie in jenem der JP 01-301166, sphärisch Partikel als mimetischer Ersatz für bestimmte Zellen (Blutplättchen bzw. Erythrozyten) des Blutes vorgesehen sind.

Aufgabe der vorliegenden Erfindung ist es, einen Kontroll- oder Kalibrierstandard für Meßgerät zur optischen Bestimmung der Hämoglobinkonzentration vorzuschlagen, welcher in Systemen mit optischen Sensoren anwendbar ist, wobei die Handhabung der Standards sowie deren Herstellung vereinfacht werden soll.

Diese Aufgabe wird erfindungsgemäß dadurch gelöst, daß die Streukörper zur Gewinnung eines der Hämoglobinkonzentration entsprechenden Meßwertes dienen und einen mittleren Durchmesser < 0,6 µm, vorzugsweise ca. 0,3 µm und eine Volumskonzentration < 0,3%, aufweisen. Überraschenderweise hat sich gezeigt, daß mit Streukörpern mit einem mittleren Durchmesser < 0,6 µm, insbesondere in Streulichtgeometrien, reproduzierbare Meßwerte erzielt werden können, welche sich für die Kalibrierung bzw. Qualitätskontrolle für Meßgeräte zur optischen Bestimmung der Hämoglobinkonzentration eignen. Aufgrund der geringen Teilchengröße werden diese durch thermische Molekularbewegung zeitlich im wesentlichen unbegrenzt in Suspension gehalten, ohne die Teilchen oder deren Oberfläche modifizieren zu müssen. Der Standard bleibt somit ohne weitere Maßnahmen homogen und ist mit optischen Systemen und Sensoren voll verträglich. Derartige Streukörper können auch ein optisches Signal ergeben, welches sich zur Qualitätskontrolle oder Kalibrierung der Sauerstoffsättigung (SO₂%) eignet.

Streukörper mit den benötigten Eigenschaften werden in unterschiedlichen Durchmessern und Materialien, beispielsweise von den Firmen PROLABO, 45300 Pithiviers, Frankreich; BANGS LABS, Fishers, IN 46038 USA; DYNO PARTICLES AS, N-2001 Lillestrom, Norwegen oder auch POLYSCIENCES INC. Warrington, PA 1876, USA, hergestellt und angeboten.

Erfindungsgemäß können die Streukörper aus im wesentlichen sphärischen Partikeln aus Polystyrol, Polyvinyltoluol, Styrolbutatien-Copolymeren, Polydivinylbenzen, Polyolefin, Polymethylmethacrylat, Polyacrylat oder Latex bestehen, wobei die spezifische Dichte der Elektrolytlösung im wesentlichen an jene der Streukörper angepaßt werden kann. Da die Sedimentationsrate von Partikeln neben deren Durchmesser (quadratische Abhängigkeit) auch von der Differenz der Dichte zwischen Partikel und Trägerflüssigkeit abhängt, können besonders stabile Lösungen erzielt werden, wenn man die Dichte der Elektrolytlösung an jene des verwendeten Polymers für die Streukörper anpaßt.

Zur Erhöhung der Sauerstofflöslichkeit und zur Anpassung der Dichte der Elektrolytlösung an jene der Streukörper kann der Kontroll- oder Kalibrierstandard wasserlösliche Polymere, insbesondere Polyvinylalkohol (PVA), Polyvinylpyrrolidon (PVP) oder Polyethylenglykol (PEG) oder mehrwertige Alkohole, insbesondere Glyzerin, Propylenglykol und Ethylenglykol in Konzentrationen bis 90 Vol% aufweisen. Die Verwendung vom löslichen Polymeren in Kontrollflüssigkeiten für Blutgasanalysatoren wird beispielsweise in der US 4,945,062 A beschrieben. Im Sinne der vorliegenden Erfindung können allerdings die dort beschriebenen Konzentrationen auch überschritten werden. Beispielsweise kann die Sauerstoff löslichkeit durch Zusatz von > 5 Vol% (bevorzugt > 10 Vol%) der genannten Polymere signifikant erhöht werden, was eine Verbesserung der Stabilität der Flüssigkeiten zur Folge hat. Neben den genannten Polymeren eignen sich auch monomere Stoffe, bevorzugt mehrwertige Alkohole, wie Glyzerin und Ethylenglykol dafür, die Sauerstofflöslichkeit zu erhöhen. Speziell in Blutgaskontrollflüssigkeiten können Konzentrationen > 50 Vol% eingesetzt werden. Die Konzentrationsobergrenze hängt von der Handhabbarkeit der Lösungen ab, die meist durch die Viskosität bei hohen Prozentsätzen der Zusätze bestimmt wird. Beispielsweise weisen 90% Ethylenglykol in Wasser eine Viskosität von 14cp (bei 20°C) auf, wobei Lösungen dieser Art noch als verwendbar einzustufen sind.

Allen diesen Lösungen ist gemeinsam, daß die Dichte erhöht wird, sodaß Werte zwischen 1,000 (Wasser) und 1,11 g/ml (90% Ethylenglykol in Wasser) auftreten. Der Elektrolyt- und Puffergehalt im interessierenden Bereich beeinflußt hingegen die Dichte des Standards in geringerem Ausmaß (maximal 0,015 g/ml).

Da Streukörper mit Materialien unterschiedlicher Dichte im Handel erhältlich sind, kann die Dichte zwischen Lösung und Streukörper durch den Einsatz der am besten geeigneten Streukörpermaterialien abgestimmt werden. Eine Feinabstimmung kann durch Variation des Gehaltes an löslichen Polymeren oder mehrwertigen monomeren Alkoholen erfolgen.

In einer Weiterbildung der Erfindung kann an die Oberfläche oder das Innere der Streukörper ein Absorber für Strahlung im Wellenlängenbereich zwischen 600 und 1200 nm gebunden sein. Derartige Absorber sind beispielsweise die IR-Absorber der Fa. EXCITON, INC. P. O. Box 31126 Dayton, Ohio 45437-0126 IRA 980, IRA 866 und IRA 850. Bei IRA 980 (Tris[4-(diethylamino)phenyl]ammoniumyl hexafluorantimonat(1-) handelt es sich um einen hydrophoben, wasserunlöslichen Farbstoff, welcher in die Streukörper eingebracht werden kann. Gleiches gilt für IRA 866 und IRA 850, welche ebenfalls gut in die Streukörper eingebracht werden können. Letztere sind hydrophober als IRA 980. Durch den Zusatz eines Absorbers kann das Absorptionsspektrum des Standards für unterschiedliche Meßwellenlängen besser an jenes einer Blutprobe mit unterschiedlichen Hämoglobinkonzentrationen bzw. Sauerstoffsättigungen angepaßt werden.

Weiters ist es möglich, bereits gefärbte Streukörper zu verwenden (beispielsweise von BANGS LABS, Fishers, IN 46038, USA), welche Absorptionsmaxima bei 634, 740 und 830 nm aufweisen. Derartige gefärbte Streukörper eignen sich besonders gut für Standards, welche zur Kalibrierung bzw. Qualitätskontrolle der Sauerstoffsättigung herangezogen werden. Durch die Bindung des Farbstoffes an die Oberfläche oder das Innere der Streukörper kann eine Beeinträchtigung der optischen Sensoren der zu kalibrierenden Meßgeräte vermieden werden.

Um eine Aggregation der Streukörper zu vermeiden, kann der Kontroll- oder Kalibrierstandard erfindungsgemäß ein Tensid, vorzugsweise ein nichtionisches Tensid aus der Gruppe der Polyoxyethylen-alkylether, vorzugsweise einen Polyoxyethylenhexadecylether oder der Alkylphenyl-polyoxyethylenether, vorzugsweise einen Octylphenyl-polyoxyethylenether aufweisen.

Viele Meßgeräte bzw. Meßsysteme zur Bestimmung von Blutparametern enthalten neben den Meßsystemen zur Bestimmung der Hämoglobinkonzentration und der Sauerstoffsättigung Sensoren zur Bestimmung der Blutgasparameter (pH, PCO₂, PO₂) sowie Sensoren für die Messung unterschiedlicher Ionenkonzentrationen (z. B. Na, K, Ca, Mg, Cl). Die oben beschriebenen Kontroll- und Kalibrierstandards sind auch für die Qualitätskontrolle bzw. Kalibrierung derartiger Geräte geeignet, wenn erfindungsgemäß die Elektrolytlösung einen Puffer zur Bereitstellung eines pH-Wertes zwischen 6,7 und 7,9 vorzugsweise zwischen 7,1 und 7,7 aufweist, sowie die Werte für PCO₂ zwischen 1.33 und 13.3 kPa (10 und 100 mmHg), vorzugsweise zwischen 2.67 und 10.67 kPa (20 und 80 mmHg) und für PO₂ zwischen 2.00 und 53.3 kPa (15 und 400 mmHg), vorzugsweise zwischen 6.00 und 21.3 kPa (45 und 160 mmHg) liegen.

Weiters ist vorgesehen, daß die Elektrolytlösung zumindest ein Ion aus der Gruppe bestehend aus Ca²⁺-, Na⁺-, K⁺-, Mg²⁺- und Cl⁻-Ionen in den für die Messung der entsprechenden Ionenkonzentrationen im Blut bekannten Konzentrationswerten enthält.

Erfindungsgemäß ist vorgesehen, daß im Standard als Puffer ein Good's Puffer, vorzugsweise HEPES, MOPS oder TES enthalten ist.

Der erfindungsgemäße Standard kann beispielsweise als Serie von drei Lösungen vorliegen, wobei Level 1 niedrige, Level 2 normale und Level 3 hohe Konzentrationswerte für die einzelnen Parameter aufweist. Insbesondere können die drei Lösungen folgende Zusammensetzung aufweisen:

| ANALYT Einheit | | Level 1 Bereich | | Level 2 Bereich | | Level 3 Bereich | |
|---|---|---|---|---|---|---|---|
| | | Mittelwerte +/-Abweich. | | Mittelwerte | +/-Abweich | Mittelwerte | +/-Abweich |
| pH | pH un. | 7.14-7.20 | 0.003 | 7.40-7.44 | 0.003 | 7.58-7.64 | 0.003 |
| PCO₂ | kPa | 8.67 - 10.0 | 0.1 | 5.33 - 6.13 | 0.07 | 2.67 - 3.73 | 0.07 |
| | mmHg | 65-75 | 0.75 | 40-46 | 0.50 | 20-28 | 0.50 |
| PO₂ | kPa | 8.67 - 10.0 | 0.2 | 13.47 - 14.80 | 0.2 | 17.33 - 20,00 | 0.23 |
| | mmHg | 65-75 | 1.5 | 101-111 | 1.5 | 130-150 | 1.7 |
| Na⁺ | mmol/L | 115-125 | 1.5 | 135-145 | 1.5 | 150-180 | 1.5 |
| K⁺ | mmol/L | 2.5-3.5 | 0.09 | 4.5-5.5 | 0.09 | 5.5-6.5 | 0.09 |
| Cl⁻ | mmol/L | 81-91 | 1.0 | 98-108 | 1.0 | 116-126 | 1.0 |
| Ca²⁺ | mmol/L | 1.3-1.5 | 0.05 | 1.05-1.25 | 0.05 | 0.6-0.8 | 0.05 |
| Streukörper | Vol% | 0.008-0.012 | 0.001 | 0.04-0.08 | 0.005 | 0.13-0.17 | 0.01 |

Die in der obigen Tabelle angegebenen Konzentrationswerte der Streukörper (Beads) in den Leveln 1 bis 3 der Standardflüssigkeit ergeben in einem Blutgasanalysator (OPTI 2 der Fa. AVL MEDICAL INSTRUMENTS AG, Schaffhausen CH) folgende Werte für die Hämoglobinkonzentration (tHb) und die Sauerstoffsättigung (SO₂ %):
Level 1: tHb = 16 - 19g/dl ± 1g/dl; SO₂ = 80 - 86% ± 1,5%
Level 2: tHb = 12 - 15g/dl ± 1g/dl; SO₂ = 88 - 94% ± 1,5%
Level 3: tHb = 8 - llg/dl ± 1g/dl; SO₂ = 94 - 100% ± 1,5%.

Zur Realisierung der oben genannten Hämoglobinkonzentrationen und SO₂-Werte ist vorgesehen, daß die Streukörper aus nichtfunktionalisierten Polystyrol-Beads mit einem Durchmesser von 0,3 µm bestehen. Die Polystyrol-Beads weisen eine Dichte von 1,050g/ml auf und werden in einer wäßrigen Suspension mit 10% Feststoffgehalt von der Firma BANGS LAB, 9025 Fishers, IN 46038, USA angeboten.

Erfindungsgemäß kann dem Kontroll- oder Kalibrierstandard ein Konservierungsmittel aus der Gruppe der Isothiazoline, beispielsweise Proclin 300 © (ROHM AND HAAS GmbH, 60489 Frankfurt, DE) oder Mergal K9N © (RIEDEL-DE HAEN, 30918 Seelze, DE) zugesetzt sein. Diese enthalten als wirksame Stoffe 5-Chlor-2-methyl-2H-isothiazol-3-on und 2-Methyl-2H-isothiazol-3-on.

Der Standard wird in Ampullen abgefüllt, wobei die Mindestfüllmenge ca. 1,7 ml beträgt.

Ein Herstellungsbeispiel für eine erfindungsgemäße Standardlösung bestehend aus einer Serie von drei Lösungen wird nachstehend angeführt:

| | Einheit | Level 1 | Level 2 | Level 3 |
|---|---|---|---|---|
| HEPES (freie Säure) | mmol/L | 42 | 42 | 42 |
| NaOH | mmol/L | 30 | 30 | 30 |
| NaCl | mmol/L | 77 | 93 | 112 |
| NaHCO₃ | mmol/L | 12 | 19 | 22 |
| KCl | mmol/L | 3,0 | 5,0 | 6,0 |
| CaCl₂ | mmol/L | 1,6 | 1,35 | 0,8 |
| MgCl₂ | mmol/L | 1,2 | 0,75 | 0,4 |
| Proclin 300 | g/L | 0,2 | 0,2 | 0,2 |
| Beads (10 % in Wasser) | ml/L | 1,0 | 5,0 | 15,0 |
| Brij-58 | g/L | 0,05 | 0,05 | 0,05 |
| Begasung (bei 37 °C) | | Gas A | Gas B | Gas C |

Die Begasung erfolgt bei 37 °C bis folgende Gleichgewichte erreicht sind:

PO₂(gas) = PO₂(flüssig) und PCO₂(gas) = PCO₂(flüssig)

Gaszusammensetzungen:

| | | | |
|---|---|---|---|
| Gas A | 10 % (vol) CO₂, | 10 % (vol) O₂, | Rest N₂ |
| Gas B | 5,5 % (vol) CO₂, | 15 % (vol) O₂, | Rest N₂ |
| Gas C | 3,5 % (vol) CO₂, | 20 % (vol) O₂, | Rest N₂ |

## Patentansprüche

1. Kontroll- oder Kalibrierstandard für Meßgeräte zur optischen Bestimmung der Hämoglobinkonzentration in Blutproben, welcher in einer wäßrigen Elektrolytlösung dispergierte Streukörper aufweist, **dadurch gekennzeichnet,** daß die Streukörper zur Gewinnung eines der Hämoglobinkonzentration entsprechenden Meßwertes dienen und einen mittleren Durchmesser < 0,6 µm, vorzugsweise ca. 0,3 µm und eine Volumskonzentration < 0,3%, aufweisen.

2. Kontroll- oder Kalibrierstandard nach Anspruch 1, **dadurch gekennzeichnet,** daß die Streukörper aus im wesentlichen sphärischen Partikeln aus Polystyrol, Polyvinyltoluol, Styrolbutatien-Copolymeren, Polydivinylbenzen, Polyolefin, Polymethylmethacrylat, Polyacrylat oder Latex bestehen.

3. Kontroll- oder Kalibrierstandard nach Anspruch 1 oder 2, **dadurch gekennzeichnet,** daß an die Oberfläche oder das Innere der Streukörper ein Absorber für Strahlung im Wellenlängenbereich zwischen 600 und 1200 nm gebunden ist.

4. Kontroll- oder Kalibrierstandard nach einem der Ansprüche 1 bis 3, **dadurch gekennzeichnet,** daß die spezifische Dichte der Elektrolytlösung im wesentlichen jener der Streukörper entspricht.

5. Kontroll- oder Kalibrierstandard nach Anspruch 4, **dadurch gekennzeichnet,** daß die Elektrolytlösung zur Erhöhung der Sauerstofflöslichkeit und/oder zur Anpassung der Dichte an jene der Streukörper wasserlösliche Polymere, insbesondere Polyvinylalkohol (PVA), Polyvinylpyrrolidon (PVP) oder Polyethylenglykol (PEG) oder mehrwertige Alkohole, insbesondere Glyzerin, Propylenglykol und Ethylenglykol in Konzentrationen bis 90 Vol% aufweist.

6. Kontroll- oder Kalibrierstandard nach einem der Ansprüche 1 bis 5, **dadurch gekennzeichnet,** daß der Standard ein Tensid, vorzugsweise ein nichtionisches Tensid aus der Gruppe der Polyoxyethylen-alkylether, vorzugsweise einen Polyoxyethylen-hexadecylether oder der Alkylphenyl-polyoxyethylenether, vorzugsweise einen Octylphenyl-polyoxyethylenether aufweist

7. Kontroll- oder Kalibrierstandard nach einem der Ansprüche 1 bis 6, welcher zusätzlich für die Qualitätskontrolle und/oder Kalibrierung von Blutgasmeßsystemen geeignet ist, **dadurch gekennzeichnet,** daß die Elektrolytlösung einen Puffer zur Bereitstellung eines pH-Wertes zwischen 6,7 und 7,9 vorzugsweise zwischen 7,1 und 7,7 aufweist, sowie daß die Werte für PCO₂ zwischen 1.33 und 13.3 kPa (10 und 100 mmHg), vorzugsweise zwischen 2.67 und 10.67 kPa (20 und 80 mmHg) und für PO₂ zwischen 2.00 und 53.3 kPa (15 und 400 mmHg), vorzugsweise zwischen 6.00 und 21.3 kPa (45 und 160 mmHg) liegen.

8. Kontroll- oder Kalibrierstandard nach Anspruch 7, **dadurch gekennzeichnet,** daß die Elektrolytlösung zumindest ein Ion aus der Gruppe bestehend aus Ca²⁺-, Na⁺-, K⁺-, Mg²⁺- und Cl⁻-Ionen in den für die Messung der entsprechenden Ionenkonzentrationen im Blut bekannten Konzentrationswerten enthält.

9. Kontroll- oder Kalibrierstandard nach Anspruch 7 oder 8, **dadurch gekennzeichnet,** daß als Puffer ein Good's Puffer, vorzugsweise HEPES, MOPS oder TES enthalten ist.

10. Kontroll- oder Kalibrierstandard nach einem der Ansprüche 7 bis 9, **dadurch gekennzeichnet,** daß der Standard eine Serie von drei Lösungen (Level 1, Level 2 und Level 3) der folgenden Zusammensetzung umfaßt:
| ANALYT Einheit | | Level 1 Bereich | | Level 2 Bereich | | Level 3 Bereich | |
|---|---|---|---|---|---|---|---|
| | | Mittelwerte | +/-Abweich | Mittelwerte | +/-Abweich | Mittelwerte | +/-Abweich |
| pH | pH un. | 7.14-7.20 | 0.003 | 7.40-7.44 | 0.003 | 7.58-7.64 | 0.003 |
| PCO₂ | kPa | 8.67 - 10.0 | 0.1 | 5.33 - 6.13 | 0.07 | 2.67 - 3.73 | 0.07 |
| | mmHg | 65-75 | 0.75 | 40-46 | 0.50 | 20-28 | 0.50 |
| PO₂ | kPa | 8.67 - 10.0 | 0.2 | 13.47 - 14.80 | 0.2 | 17.33 - 20,00 | 0.23 |
| | mmHg | 65-75 | 1.5 | 101-111 | 1.5 | 130-150 | 1.7 |
| Na⁺ | mmol/L | 115-125 | 1.5 | 135-145 | 1.5 | 150-180 | 1.5 |
| K⁺ | mmol/L | 2.5-3.5 | 0.09 | 4.5-5.5 | 0.09 | 5.5-6.5 | 0.09 |
| Cl⁻ | mmol/L | 81-91 | 1.0 | 98-108 | 1.0 | 116-126 | 1.0 |
| Ca²⁺ | mmol/L | 1.3-1.5 | 0.05 | 1.05-1.25 | 0.05 | 0.6-0.8 | 0.05 |
| Streukörper | Vol% | 0.008-0.012 | 0.001 | 0.04-0.08 | 0.005 | 0.13-0.17 | 0.01 |

11. Kontroll- oder Kalibrierstandard nach Anspruch 10, **dadurch gekennzeichnet,** daß die Streukörper aus nichtfunktionalisierten Polystyrol-Beads mit einem Durchmesser von 0,3 µm bestehen.

12. Kontroll- oder Kalibrierstandard nach einem der Ansprüche 1 bis 11, **dadurch gekennzeichnet,** daß der Standard ein Konservierungsmittel, beispielsweise aus der Gruppe der Isothiazoline aufweist.

## Claims

1. Control or calibration standard for use with instruments for optical measurement of the haemoglobin concentration in blood samples, which is comprised of scattering particles dispersed in aqueous electrolyte solution, **characterized in that** said particles are used to obtain a measured value corresponding to haemoglobin concentration and have a mean diameter of < 0.6 µm, and preferably about 0.3 µm, and a volume concentration < 0.3%.

2. Control or calibration standard according to claim 1, **characterized in that** said scattering particles consist of substantially spherical particles made of polystyrene, polyvinyl toluene, styrene butadiene copolymers, polydivinyl benzene, polyolefin, polymethyl methacrylate, polyacrylate, or latex.

3. Control or calibration standard according to claim 1 or 2, **characterized in that** a radiation absorber for wavelengths in the 600-1,200 nm range is bound on the surface or in the interior of the scattering particles.

4. Control or calibration standard according to any of claims 1 to 3, **characterized in that** the mass density of the electrolyte solution substantially corresponds to that of the scattering particles.

5. Control or calibration standard according to claim 4, **characterized in that** the electrolyte solution contains water-soluble polymers, especially polyvinyl alcohol (PVA), polyvinyl pyrrolidone (PVP), or polyethylene glycol (PEG), or polyvalent alcohols, especially glycerol, propylene glycol and ethylene glycol, in concentrations of up to 90 percent by volume, in order to increase oxygen solubility and/or to match the density of the electrolyte solution with that of the scattering particles.

6. Control or calibration standard according to any of claims 1 to 5, **characterized in that** the standard contains a surfactant, i.e., preferably a non-ionic tenside from the group of polyoxyethylene alkyl ethers, and preferably a polyoxyethylene hexadecylic ether, or of alkylphenyl polyoxyethylene ethers, and preferably an octylphenyl polyoxyethylene ether.

7. Control or calibration standard according to any of claims 1 to 6, which is additionally suited for the quality control and/or calibration of blood gas measuring systems, **characterized in that** the electrolyte solution contains a buffer providing a pH of 6.7 to 7.9, and preferably 7.1 to 7.7, and further that the values for PCO₂ are between 1.33 and 13.3 kPa (10 and 100 mmHg), and preferably between 2.67 and 10.67 kPa (20 and 80 mmHg), and for P02 between 2.00 and 53.3 kPa (15 and 400 mmHg), and preferably between 6.00 and 21.3 kPa (45 and 160 mmHg).

8. Control or calibration standard according to claim 7, **characterized in that** the electrolyte solution contains at least one ion from the group of Ca²⁺, Na⁺, K⁺, Mg²⁺, and Cl⁻ ions at concentrations corresponding to the ion concentrations found in the blood.

9. Control or calibration standard according to claim 7 or 8, **characterized in that** the standard contains a Good's buffer as a buffer material, i.e., preferably HEPES, MOPS, or TES.

10. Control or calibration standard according to any of claims 7 to 9, **characterized in that** the standard comprises a series of three solutions (Level 1, Level 2, Level 3) of the following composition:
| ANALYTE | UNITS | Level 1 RANGE | | Level 2 RANGE | | Level 3 RANGE | |
|---|---|---|---|---|---|---|---|
| | | MEAN VALUES | +/-VAR | MEAN VALUES | +/-VAR | MEAN VALUES | +/-VAR |
| pH | pH un. | 7.14-7.20 | 0.003 | 7.40-7.44 | 0.003 | 7.58-7.64 | 0.003 |
| PCO₂ | kPa | 8.67-10.0 | 0.1 | 5.33-6.13 | 0.07 | 2.67-3.73 | 0.07 |
| | mmHg | 65-75 | 0.75 | 40-46 | 0.50 | 20-28 | 0.50 |
| PO₂ | kPa | 8.67-10.0 | 0.2 | 13.47-14.80 | 0.2 | 17.33-20.00 | 0.23 |
| | mmHg | 65-75 | 1.5 | 101-111 | 1.5 | 130-150 | 1.7 |
| Na⁺ | mmol/L | 115-125 | 1.5 | 135-145 | 1.5 | 150-180 | 1.5 |
| K⁺ | mmol/L | 2.5-3.5 | 0.09 | 4.5-5.5 | 0.09 | 5.5-6.5 | 0.09 |
| Cl⁻ | mmol/L | 81-91 | 1.0 | 98-108 | 1.0 | 116-126 | 1.0 |
| Ca²⁺ | mmol/L | 1.3-1.5 | 0.05 | 1.05-1.25 | 0.05 | 0.6-0.8 | 0.05 |
| beads | vol% | 0.008-0.012 | 0.001 | 0.04-0.08 | 0.005 | 0.13-0.17 | 0.01 |

11. Control or calibration standard according to claim 10, **characterized in that** the scattering particles are non-functionalized polystyrene beads of a diameter of 0.3 µm.

12. Control or calibration standard according to any of claims 1 to 11, **characterized in that** the standard contains a preservative, for example from the group of isothiazolines.

## Revendications

1. Standard de contrôle ou d'étalonnage pour des appareils de mesure, pour la détermination optique de la concentration en hémoglobine dans des échantillons sanguins, présentant des corps de dispersion, dispersés dans une solution aqueuse d'électrolyte, caractérisé par le fait que les corps de dispersion servent à l'obtention d'une valeur de mesure correspondant à la concentration en hémoglobine et présentent un diamètre moyen < 0,6 µm, de préférence d'environ 0,3 µm, et une concentration en volume < 0,3 %.

2. Standard de contrôle ou d'étalonnage selon la revendication 1, caractérisé par le fait que les corps de dispersion sont constitués de particules pratiquement sphériques en polystyrène, polyvinyltoluène, copolymères de styrène-butadiène, polydivinylbenzène, polyoléfine, polyméthylméthacrylate, polyacrylate ou latex.

3. Standard de contrôle ou d'étalonnage selon la revendication 1 ou 2, caractérisé par le fait qu'un absorbeur de rayonnement, agissant dans la plage de longueurs d'onde comprise entre 600 et 1200 nm, est lié à la surface ou à l'intérieur des corps de dispersion.

4. Standard de contrôle ou d'étalonnage selon l'une des revendications 1 à 3, caractérisé par le fait que la masse volumique de la solution d'électrolyte correspond sensiblement à celle des corps de dispersion.

5. Standard de contrôle ou d'étalonnage selon la revendication 4, caractérisé par le fait que, pour augmenter la solubilité de l'oxygène et/ou pour obtenir l'adaptation de la masse volumique à celle des corps de dispersion, la solution électrolyte présente des polymères solubles dans l'eau, en particulier de l'alcool polyvinylique (PVA), de la polyvinylpyrrolidone (PVP) ou du polyéthylène glycol (PEG), ou des alcools polyvalents, en particulier de la glycérine, du propylène glycol et de l'éthylène glycol, en des concentrations allant jusqu'à 90 % en volume.

6. Standard de contrôle ou d'étalonnage selon l'une des revendications 1 à 5, caractérisé par le fait que le standard présente un tensioactif, de préférence un tensioactif non ionique, issu du groupe des polyoxyéthylène-alkyl éthers, de préférence un polyoxyéthylène-hexadécyl éther, ou des alkylphényl-polyoxyéthylène éthers, de préférence un octylphényl-polyoxyéthylène éther.

7. Standard de contrôle ou d'étalonnage selon l'une des revendications 1 à 6, convenant en plus pour le contrôle de qualité et/ou l'étalonnage de systèmes de mesure des gaz du sang, caractérisé par le fait que la solution d'électrolyte présente un tampon pour la préparation d'une valeur de pH comprise entre 6,7 et 7,9, de préférence entre 7,1 et 7,7, et en ce que les valeurs de PCO₂ sont situées entre 1,33 et 13,3 kPa (10 et 100 mmHg), de préférence entre 2,67 et 10,67 kPa (20 et 80 mmHg) et, pour PO₂, entre 2,00 et 53,3 kPa (15 et 400 mmHg), de préférence entre 6,00 et 21,3 kPa (45 et 160 mmHg).

8. Standard de contrôle ou d'étalonnage selon la revendication 7, caractérisé par le fait que la solution d'électrolyte contient au moins un ion issu du groupe constitué des ions Ca²⁺, Na⁺, K⁺, Mg²⁺ et Cl⁻, dans les valeurs de concentration connues pour la mesure des concentrations ioniques correspondantes dans le sang.

9. Standard de contrôle ou d'étalonnage selon la revendication 7 ou 8, caractérisé par le fait qu'un tampon de Good, de préférence un tampon HEPES, MOPS ou TES, est contenu à titre de tampon.

10. Standard de contrôle ou d'étalonnage selon l'une des revendications 7 à 9, caractérisé par le fait que le standard comprend une série de trois solutions (Level 1, Level 2 et Level 3) de compositions suivantes :
| | | Plage Level 1 | | Plage Level 2 | | Plage Level 3 | |
|---|---|---|---|---|---|---|---|
| COMPOSANT | Unité | Valeurs moyennes | Ecart ± | Valeurs moyennes | Ecart ± | Valeurs moyennes | Ecart ± |
| pH | un. pH | 7,14-7,20 | 0,003 | 7,40-7,44 | 0,003 | 7,58-7,64 | 0,003 |
| PCO₂ | kPa | 8,67-10,0 | 0,1 | 5,33-6,13 | 0,07 | 2,67-3,73 | 0,07 |
| | mmHg | 65-75 | 0,75 | 40-46 | 0,50 | 20-28 | 0,50 |
| PO₂ | kPa | 8,67-10,0 | 0,2 | 13,47-14,80 | 0,2 | 17,33-20,00 | 0,23 |
| | mmHg | 65-75 | 1,5 | 101-111 | 1,5 | 130-150 | 1,7 |
| Na⁺ | mmol/L | 115-125 | 1,5 | 135-145 | 1,5 | 150-180 | 1,5 |
| K⁺ | mmol/L | 2,5-3,5 | 0,09 | 4,5-5,5 | 0,09 | 5,5-6,5 | 0,09 |
| Cl⁻ | mmol/L | 81-91 | 1,0 | 98-108 | 1,0 | 116-126 | 1,0 |
| Ca²⁺ | mmol/L | 1,3-1,5 | 0,05 | 1,05-1,25 | 0,05 | 0,6-0,8 | 0,05 |
| Corps de dispersion | %.vol | 0,008-0,012 | 0,001 | 0,04-0,08 | 0,005 | 0,13-0,17 | 0,01 |

11. Standard de contrôle ou d'étalonnage selon la revendication 10, caractérisé par le fait que les corps de dispersion sont constitués de perles de polystyrène non fonctionnalisées, d'un diamètre de 0,3 µm.

12. Standard de contrôle ou d'étalonnage selon l'une des revendications 1 à 11, caractérisé par le fait que le standard présente un agent de conservation, de préférence issu du groupe de l'isothiazoline.
